# EUROPÄISCHE PATENTSCHRIFT

(11) **EP 2 097 014 B1**
(45) Veröffentlichungstag und Bekanntmachung des Hinweises auf die Patenterteilung: **17.07.2019**
(21) Anmeldenummer: 07847386.5
(22) Anmeldetag: 27.11.2007
(51) Int. Cl.: A61B 17/16

(54) **CHIRURGISCHES GLEICHSTROMELEKTROWERKZEUG MIT CHIRURGISCHEM SCHALTNETZTEIL**
SURGICAL DC POWER TOOL WITH SURGICAL SWITCHED-MODE POWER SUPPLY
APPAREIL ÉLECTRIQUE CHIRURGICAL À COURANT CONTINU AVEC ALIMENTATION CHIRURGICALE À DECOUPAGE

(30) Priorität: 07.12.2006 DE 102006058867
(43) Veröffentlichungstag der Anmeldung: 09.09.2009
(73) Patentinhaber: Aesculap AG, 78532 Tuttlingen (DE)
(72) Erfinder: KAHLER, Thomas, 78606 Seitingen-Oberflacht (DE); HOEGERLE, Roland, 78532 Tuttlingen (DE)
(74) Vertreter: Hoeger, Stellrecht & Partner Patentanwälte mbB
(86) Internationale Anmeldenummer: PCT/EP2007/062859
(87) Internationale Veröffentlichungsnummer: WO 2008/068160

(56) Entgegenhaltungen:
- WO-A-03/075442
- WO-A-03/079525
- WO-A1-88/02242
- DE-A1-102005 015 654
- DE-U1- 8 907 055
- US-A1- 2003 220 638
- US-A1- 2005 096 661
- US-A1- 2006 217 729
- US-B1- 6 223 077

## Beschreibung

Die vorliegende Erfindung betrifft ein chirurgisches Gleichstromelektrowerkzeug mit einem Gehäuse, einem im Gehäuse aufgenommenen Gleichstromelektroverbraucher und einer Schnittstelle für eine netzunabhängige Energiequelle, wobei das Gehäuse eine Energieversorgungsaufnahme für die netzunabhängige Energiequelle aufweist.

Chirurgische Gleichstromelektrowerkzeuge werden beispielsweise in Form von batterie- oder akkubetriebenen chirurgischen Antriebsmaschinen in der Chirurgie eingesetzt. Derartige Antriebsmaschinen, zum Beispiel Bohrmaschinen oder Sägen haben den Nachteil, dass jede Batterie beziehungsweise jeder Ackumulator nur eine begrenzte Energiemenge speichern kann. Bei langwierigen chirurgischen Eingriffen kann es daher vorkommen, dass die Batterie vollständig entleert wird und wieder aufgeladen werden muss. Das Laden der Batterie benötigt jedoch Zeit. Ferner ist zum Laden eine Ladegerät und/oder eine Wechselbatterie erforderlich, um die weitere Einsatzfähigkeit der Antriebsmaschine sicherzustellen. Zudem ist zu berücksichtigen, dass das Laden der Batterie beziehungsweise ein Auswechseln derselben unter sterilen Bedingungen erfolgen muss.

Aus der DE 10 2005 015 654 A1 ist ein Akku-Schaltnetzteil-Ladegerät bekannt. In der US 2005/0096661 A1 ist eine isolierte Batteriepackung beschrieben. Eine Energieversorgung zur Identifikation und Steuerung elektro-chirurgischer Werkzeuge ist in der WO 03/079525 A2 offenbart. Aus der WO 03/ 075442 A1 ist ein Schaltnetzteil, insbesondere für medizinische Vorrichtungen, bekannt. Die US 6,223,077 B1 betrifft eine automatische Leistungsschaltung in einem Defibrillator. Eine voll sterilisierbare Akkubohrmaschine ist in der DE 89 07 055 U1 beschrieben. Ferner ist aus der US 2004/0054365 A1 ein elektrochirurgisches System bekannt. Die US 2003/0220638 A1 offenbart eine Vorrichtung zur Versorgung eines Elektrostifts mit elektrischer Energie. In der WO 88/02242 A1 ist eine chirurgische Knochenbohrmaschine beschrieben. Ein chirurgisches Gerät und Werkzeuge für dieses sind aus der US 2006/0217729 A1 bekannt.

Es ist daher Aufgabe der vorliegenden Erfindung, ein chirurgisches Gleichstromelektrowerkzeug so zu verbessern, dass es gegebenenfalls, insbesondere unter sterilen Bedingungen, unter Nutzung herkömmlicher Wechselstromspannungsversorgungen betrieben werden kann.

Diese Aufgabe wird bei einem chirurgischen Gleichstromelektrowerkzeug der eingangs beschriebenen Art erfindungsgemäß gelöst durch ein chirurgisches Schaltnetzteil, welches eine Schaltnetzteilschaltungsanordnung, ein Schaltnetzteilgehäuse und eine im Schaltnetzteilgehäuse ausgebildete Schaltungsaufnahme zum Aufnehmen der Schaltnetzteilschaltungsanordnung umfasst, und dass das Schaltnetzteilgehäuse eine Schaltnetzteilschnittstelle aufweist, die derart ausgebildet ist, dass sie mit der für die netzunabhängige Energiequelle vorgesehenen Schnittstelle des Gleichstromelektrowerkzeugs in Eingriff bringbar ist, dass das chirurgische Schaltnetzteil derart ausgebildet ist, dass es in die Energieversorgungsaufnahme vollständig einführbar und in dieser festlegbar ist, dass mindestens eine Dichtung vorgesehen ist zum fluiddichten Abdichten der Schaltungsaufnahme und dass die mindestens eine Dichtung derart angeordnet ist, dass sie das Schaltnetzteilgehäuse relativ zum Gehäuse oder der Energieversorgungsaufnahme des Gehäuses abdichtet.

Das chirurgische Schaltnetzteil ermöglicht es, das chirurgische Gleichstromelektrowerkzeug anstatt mit einer Batterie mit einer Wechselspannungs- beziehungsweise Wechselstromenergieversorgung zu verbinden und so mit Energie zu versorgen. Insbesondere kann das chirurgische Schaltnetzteil derart ausgebildet sein, dass es mit einem jeweils landesüblichen Stromnetz verbindbar ist, beispielsweise einem in Europa üblichen Stromnetz mit einer Wechselspannung von 230 V bei einer Wechselspannungsfrequenz zwischen 50 und 60 Hz. Alternativ ist es selbstverständlich denkbar, auch 110 V-Wechselspannungsnetze mit dem Schaltnetzteil zum Betrieb eines chirurgischen Gleichstromelektrowerkzeugs zu nutzen. Das chirurgische Schaltnetzteil hat ferner den Vorteil, dass insbesondere bei chirurgischen Eingriffen, für die eine einzige Batterieladung nicht ausreichend ist, das benötigte chirurgische Gleichstromelektrowerkzeug mit dem chirurgischen Schaltnetzteil in Eingriff gebracht und/oder verbunden werden kann, wodurch es beliebig lange betreibbar und ein Austausch der Energieversorgung nicht erforderlich ist. Ferner sei insbesondere darauf hingewiesen, dass unter einem chirurgischen Schaltnetzteil im Sinne der Erfindung kein separates Steuergerät zu verstehen ist, welches beispielsweise über eine Kabelverbindung mit dem chirurgischen Gleichstromelektrowerkzeug verbunden werden kann. Das chirurgische Schaltnetzteil soll vielmehr direkt mit dem Gleichstromelektrowerkzeug beziehungsweise mit der Schnittstelle für eine netzunabhängige Energiequelle in Eingriff bringbar und/ oder verbindbar sein. Das erfindungsgemäß vorgeschlagene Gleichstromelektrowerkzeug ermöglicht es, anstelle einer netzunabhängigen Energieversorgung ein chirurgisches Schaltnetzteil zu nutzen, welches eine Energieversorgung des chirurgischen Gleichstromelektrowerkzeugs durch Verbinden mit einem vorhandenen Stromnetz ermöglicht. Um zu verhindern, dass Keime aus der Schaltungsaufnahme herausgelangen können, ist es günstig, dass mindestens eine Dichtung vorgesehen ist zum fluiddichten Abdichten der Schaltungsaufnahme. Günstigerweise ist die mindestens eine Dichtung derart angeordnet, dass sie das Schaltnetzteilgehäuse relativ zum Gehäuse des chirurgischen Gleichstromelektrowerkzeugs oder zur Energieversorgungsaufnahme des Gehäuses abdichtet. Wie bereits dargelegt, ermöglicht eine derartige Ausgestaltung, auf das Verschließen des Schaltnetzteilgehäuses mit einem Schaltnetzteilgehäusedeckel zu verzichten. Dadurch wird der Aufbau des chirurgischen Schaltnetzteils sowohl vereinfacht als auch kostengünstiger. Gemäß der Erfindung ist vorgesehen, dass das Gehäuse eine Energieversorgungsaufnahme für die netzunabhängige Energiequelle aufweist und dass das chirurgische Schaltnetzteil derart ausgebildet ist, dass es in die Energieversorgungsaufnahme mindestens teilweise einführbar und in dieser festlegbar ist. Je nach Einsatzzweck und Verwendung des chirurgischen Gleichstromelektrowerkzeugs kann die Energieversorgungsaufnahme mit einer netzunabhängigen Energiequelle, beispielsweise einer Batterie, oder dem chirurgischen Schaltnetzteil beschickt werden. Die Handhabung des chirurgischen Gleichstromelektrowerkzeugs ändert sich für eine bedienende Person praktisch nicht, weil das chirurgische Schaltnetzteil derart ausgebildet ist, dass es vollständig in die Energieversorgungsaufnahme einführbar ist.

Vorteilhaft ist es, wenn die Schaltnetzteilschnittstelle derart ausgebildet ist, dass sie mit der Schnittstelle des Gleichstromelektrowerkzeugs lösbar verbindbar ist. So ist es möglich, das chirurgische Schaltnetzteil zum Beispiel zu Reinigungszwecken vom chirurgischen Gleichstromelektrowerkzeug zu lösen.

Günstig ist es, wenn die Schaltnetzteilschnittstelle derart ausgebildet ist, dass sie mit der Schnittstelle des Gleichstromelektrowerkzeugs elektrisch und/oder mechanisch verbindbar ist. Insbesondere wenn die Schaltnetzteilschnittstelle korrespondierend zur Schnittstelle des Gleichstromelektrowerkzeugs ausgebildet ist, kann auf einfache Weise eine Batterie oder ein Akkumulator, die zur Energieversorgung des Gleichstromwerkzeugs verwendet werden können, durch das chirurgische Schaltnetzteil zeitweise oder dauerhaft ersetzt werden.

Chirurgische Gleichstromelektrowerkzeuge weisen häufig eine Energieversorgungsaufnahme für eine Batterie auf, die in einigen Fällen auch verschließbar ist. Dies ermöglicht es insbesondere, eine nicht sterilisierte Batterie in die Energieversorgungsaufnahme einzuführen, wobei trotzdem eine Sterilität des chirurgischen Gleichstromelektrowerkzeugs sichergestellt werden kann. Um die netzunabhängige Energiequelle auf einfache Weise zu ersetzen, ist es vorteilhaft, wenn das chirurgische Schaltnetzteil derart ausgebildet ist, dass es in eine für eine netzunabhängige Energiequelle vorgesehene Energieversorgungsaufnahme des Gleichstromelektrowerkzeugs mindestens teilweise einführbar und in oder an dieser festlegbar ist. Dadurch ergibt sich insgesamt ein kompakter Aufbau des chirurgischen Gleichstromelektrowerkzeugs, da die Ausgestaltung des chirurgischen Schaltnetzteils, wenn überhaupt, nur zu einer geringfügigen Vergrößerung des Gleichstromelektrowerkzeugs führt.

Die Baugröße des chirurgischen Gleichstromelektrowerkzeugs lässt sich im Wesentlichen erhalten, wenn das Schaltnetzteilgehäuse derart ausgebildet ist, dass es vollständig in die Energieversorgungsaufnahme des Gleichstromelektrowerkzeugs einführbar ist.

Gemäß einer bevorzugten Ausführungsform der Erfindung kann vorgesehen sein, dass das Schaltnetzteilgehäuse eine Schaltnetzteilgehäuseöffnung aufweist, durch die die Schaltnetzteilschaltungsanordnung in die Schaltungsaufnahme einführbar ist, und dass das Schaltnetzteilgehäuse einen Schaltnetzteilgehäusedeckel zum Verschließen der Schaltnetzteilgehäuseöffnung umfasst. Eine derartige Ausgestaltung ermöglicht es insbesondere, eine nicht sterilisierbare Schaltnetzteilschaltungsanordnung zu verwenden. Diese kann beispielsweise vor dem Einsatz in ein sterilisiertes Schaltnetzteilgehäuse eingeführt werden, und zwar unter sterilen Bedingungen, so dass das Schaltnetzteilgehäuse, nachdem es mit dem Schaltnetzteilgehäusedeckel verschlossen ist, nach außen vollständig steril ist und so alle Anforderungen an eine Keimfreiheit sowohl des Schaltnetzteils als auch des chirurgischen Gleichstromelektrowerkzeugs erfüllt werden können.

Vorzugsweise ist das Schaltnetzteilgehäuse dampfsterilisierbar. Dadurch kann es beispielsweise zusammen mit Werkzeugen, die vom chirurgischen Gleichstromelektrowerkzeug angetrieben werden können, aufbereitet, das heißt gereinigt und dampfsterilisiert werden.

Besonders kostengünstig wird die Herstellung des chirurgischen Schaltnetzteils, wenn das Schaltnetzteilgehäuse aus mindestens einem Kunststoff hergestellt ist, vorzugsweise einem sterilisierbarem Kunststoff. Zudem kann ein solches Schaltnetzteilgehäuse völlig unbedenklich sterilisiert und unter den in einem Operationsbereich erforderlichen sterilen Bedingungen eingesetzt werden.

Vorzugsweise ist die mindestens eine Dichtung die Schaltnetzteilgehäuseöffnung umgebend am Schaltnetzteilgehäuse angeordnet. Auf diese Weise ist auch eine Abdichtung des Schaltnetzteilgehäuses relativ zu einem chirurgischen Gleichstromelektrowerkzeug möglich, auch dann, wenn kein Schaltnetzteilgehäusedeckel zum Verschließen der Schaltnetzteilgehäuseöffnung vorgesehen ist. Die Dichtung dichtet in diesem Fall das Schaltnetzteilgehäuse direkt relativ zum chirurgischen Gleichstromelektrowerkzeug ab, beispielsweise relativ zu einer Energieversorgungsaufnahme desselben.

Günstig ist es, wenn die mindestens eine Dichtung derart angeordnet ist, dass sie das Schaltnetzteilgehäuse und den Schaltnetzteilgehäusedeckel relativ zueinander abdichtet, wenn der Schaltnetzteilgehäusedeckel die Schaltnetzteilgehäuseöffnung verschließt. Bei dieser Ausgestaltung ist es möglich, das sterile Schaltnetzteilgehäuse mit einer unsterilen Schaltnetzteilschaltungsanordnung zu beschicken, und zwar derart, dass das Schaltnetzteilgehäuse nach Einführen der Schaltnetzteilschaltungsanordnung nach wie vor steril ist. Um zu verhindern, dass Keime aus dem Schaltnetzteilgehäuse herausgelangen können, dient das Verschließen und Abdichten der Schaltnetzteilgehäuseöffnung mittels des Schaltnetzteilgehäusedeckels. Die Dichtung kann am Schaltnetzteilgehäusedeckel und/oder am Schaltnetzteilgehäuse angeordnet sein.

Um die Schaltnetzteilschaltungsanordnung mit dem chirurgischen Gleichstromelektrowerkzeug verbinden zu können, ist es günstig, wenn am Schaltnetzteilgehäuse erste elektrische Kontakte vorgesehen sind zur Verbindung der Schaltnetzteilschaltungsanordnung mit einem Gleichstromelektroverbraucher des chirurgischen Gleichstromelektrowerkzeugs. Insbesondere können die ersten elektrischen Kontakte derart angeordnet und ausgebildet sein, dass sie das Schaltnetzteilgehäuse durchsetzen, um die in der Schaltungsaufnahme angeordnete Schaltnetzteilschaltungsanordnung mit dem Gleichstromelektroverbraucher des chirurgischen Gleichstromelektrowerkzeugs verbinden zu können.

Vorzugsweise sind die ersten elektrischen Kontakte am Schaltnetzteilgehäusedeckel angeordnet. Durch das Verschließen des Schaltnetzteilgehäuses mit dem Schaltnetzteilgehäusedeckel können die ersten elektrischen Kontakte automatisch mit der Schaltnetzteilschaltungsanordnung beim Verschließen des Schaltnetzteilgehäuses mit dem Schaltnetzteilgehäusedeckel verbunden werden.

Gemäß einer weiteren bevorzugten Ausführungsform der Erfindung können am Schaltnetzteilgehäuse zweite elektrische Kontakte vorgesehen sein zur Verbindung mit dritten elektrischen Kontakten einer Netzanschlussleitung oder zur Verbindung mit vierten elektrischen Kontakten eines Gehäusedeckels des chirurgischen Gleichstromelektrowerkzeugs. Mit den zweiten elektrischen Kontakten kann das Schaltnetzteil direkt oder indirekt mit einer Netzanschlussleitung oder einem Kabel verbunden werden, welches eine Verbindung des chirurgischen Schaltnetzteils beziehungsweise des chirurgischen Gleichstromelektrowerkzeugs mit einer Wechselspannungs- beziehungsweise Wechselstromversorgung gestattet.

Um den Aufbau des Schaltnetzteils zu vereinfachen und die Zahl freier Kontakte zu minimieren, ist es vorteilhaft, wenn eine aus dem Schaltnetzteilgehäuse herausgeführte, mit diesem unlösbar verbundene und mit der Schaltnetzteilschaltungsanordnung elektrisch verbundene Netzanschlussleitung vorgesehen ist. Beispielsweise kann die Netzanschlussleitung zusätzlich einen entsprechenden Steckverbinder aufweisen, um das chirurgische Schaltnetzteil direkt mit einem Stromnetz zu verbinden.

Um ein Zerlegen des chirurgischen Schaltnetzteils vor der Reinigung und Sterilisierung überflüssig zu machen, ist es günstig, wenn die Schaltnetzteilschaltungsanordnung sterilisierbar ist. Das chirurgische Schaltnetzteil kann dann als Ganzes sterilisiert werden. Ein Trennen der Schaltnetzteilschaltungsanordnung vom Schaltnetzteilgehäuse ist dann nicht mehr erforderlich.

Vorzugsweise ist die Schaltnetzteilschaltungsanordnung in einen sterilisierbaren Kunststoff eingegossen. Dadurch kann die häufig temperatur- und feuchtigkeitsempfindliche Elektronik der Schaltnetzteilschaltungsanordnung geschützt werden, um eine Sterilisierung des Schaltnetzteils, insbesondere eine Dampfsterilisierung, zu ermöglichen.

Besonders einfach wird der Aufbau des chirurgischen Schaltnetzteils, wenn der Kunststoff ein Epoxidharz ist.

Der Aufbau des chirurgischen Schaltnetzteils vereinfacht sich weiter, wenn die Schaltnetzteilschaltungsanordnung und das Schaltnetzteilgehäuse unlösbar miteinander verbunden sind. Außerdem kann so ein unbeabsichtigtes Lösen der Schaltnetzteilschaltungsanordnung vom Schaltnetzteilgehäuse verhindert werden.

Günstigerweise ist die Schaltnetzteilschaltungsanordnung von einer feuchtigkeitsabweisenden Membran umgeben. Die Membran ermöglicht es, die Schaltnetzteilschaltungsanordnung insbesondere auch in feuchten Umgebungen einzusetzen. Je nach Auswahl der Membran kann sogar eine Sterilisierung der Schaltnetzteilschaltungsanordnung möglich sein.

Vorteilhaft ist es, wenn eine Gleichstromverbraucher-Steuer- und/oder Regelungsschaltung vorgesehen ist, die mindestens teilweise vom Schaltnetzteilgehäuse umgeben ist. Diese Ausgestaltung hat insbesondere den Vorteil, dass das chirurgische Gleichstromelektrowerkzeug selbst nicht mit einer Gleichstromverbraucher-Steuer- und/oder Regelungsschaltung ausgestattet sein muss. Diese ist dann Teil des chirurgischen Schaltnetzteils und wird mit diesem zusammen automatisch mit dem chirurgischen Gleichstromelektrowerkzeug verbunden. Das chirurgische Gleichstromelektrowerkzeug kann so auf einfache Weise mit anderen oder verbesserten Steuer- und/oder Regelungsschaltungen ausgestattet werden, ohne dass ein zusätzliches Zerlegen des Gleichstromelektrowerkzeugs erforderlich wäre. Zudem kann mit einem solchen chirurgischen Schaltnetzteil eine netzunabhängige Energieversorgung des chirurgischen Gleichstromelektrowerkzeugs ersetzt werden, die nicht nur eine Batterie, sondern auch eine Steuer- und/oder Regelungsschaltung umfasst.

Besonders einfach wird der Aufbau des Schaltnetzteils, wenn die Schaltnetzteilschaltungsanordnung die Gleichstromverbraucher-Steuer- und/oder Regelungsschaltung umfasst. Die Gleichstromverbraucher-Steuer- und/oder Regelungsschaltung kann somit auch als Teil der Schaltnetzteilschaltungsanordnung ausgeführt sein.

Um insbesondere eine nicht sterilisierbare oder unsterile Schaltnetzteilschaltungsanordnung in ein steriles Schaltnetzteilgehäuse einzuführen, ist es vorteilhaft, wenn eine Einführhilfe hierfür vorgesehen ist. Die Einführhilfe stellt insbesondere sicher, dass die unsterile Schaltnetzteilschaltungsanordnung mit einem Teil des Schaltnetzteilgehäuses oder des chirurgischen Gleichstromelektrowerkzeugs in Kontakt kommen kann, die unbedingt steril bleiben müssen.

Besonders einfach wird der Aufbau der Einführhilfe, wenn diese trichterförmig ausgebildet ist. Beispielsweise kann sie in Form eines trichterförmigen sterilen Folienschlauchs ausgebildet sein, der mit dem Gleichstromelektrowerkzeug, insbesondere dessen Energieversorgungsaufnahme, verbindbar ist, und durch den die unsterile Schaltnetzteilschaltungsanordnung in das Schaltnetzteilgehäuse oder die Energieversorgungsaufnahme des Gleichstromelektrowerkzeugs eingeführt werden kann.

Um das chirurgische Gleichstromelektrowerkzeug mit einem vorhandenen Stromnetz verbinden zu können, ist es vorteilhaft, wenn die Schaltnetzteilschaltungsanordnung eine Gleichrichterschaltung zum Wandeln einer Wechselspannung in eine Gleichspannung umfasst.

Gemäß einer bevorzugten Ausführungsform der Erfindung kann vorgesehen sein, dass die Schaltnetzteilschaltungsanordnung Netzanschlusskontakte zum direkten oder indirekten Verbinden mit einer Wechselspannungsenergieversorgung und Gleichstromanschlusskontakte zum direkten oder indirekten Verbinden mit einem Gleichstromelektrowerkzeug oder einem Gleichstromverbrauchers desselben aufweist. Es sind daher grundsätzlich keine weiteren Bauteile erforderlich, um das chirurgische Gleichstromelektrowerkzeug mit einem vorhandenen Stromnetz zu verbinden.

Besonders einfach wird der Aufbau des Schaltnetzteils und ein Verbinden desselben mit einem chirurgischen Gleichstromelektrowerkzeug, wenn die ersten elektrischen Kontakte, die zweiten elektrischen Kontakte, die dritten elektrischen Kontakte, die vierten elektrischen Kontakte, die Netzanschlusskontakte und/oder die Gleichstromanschlusskontakte in Form von elektrischen Steckverbindern ausgebildet sind. Die einzelnen Teile des Schaltnetzteils können so auf einfache Weise zusammengesteckt werden, ebenso das Schaltnetzteil zum Verbinden mit dem chirurgischen Gleichstromelektrowerkzeug.

Um gegebenenfalls das chirurgische Schaltnetzteil wieder durch eine netzunabhängige Energieversorgung auswechseln zu können, ist es vorteilhaft, wenn die Schaltnetzteilschnittstelle derart ausgebildet ist, dass sie mit der Schnittstelle des Gleichstromelektrowerkzeugs lösbar verbindbar ist.

Vorzugsweise ist die Schaltnetzteilschnittstelle derart ausgebildet, dass sie mit der Schnittstelle des Gleichstromelektrowerkzeugs elektrisch und/oder mechanisch verbindbar ist. Nach Verbinden des chirurgischen Gleichstromelektrowerkzeugs mit dem Schaltnetzteil ändert sich die Handhabung des Gleichstromelektrowerkzeugs für eine bedienende Person praktisch nicht im Vergleich zur Verwendung einer netzunabhängigen Energieversorgung in Verbindung mit dem chirurgischen Gleichstromelektrowerkzeug. Einzig eine elektrische Leitung zur Verbindung des Schaltnetzteils des chirurgischen Gleichstromelektrowerkzeugs mit dem vorhandenen Stromnetz ist zusätzlich vorhanden.

Vorzugsweise ist die Schnittstelle an oder in der Energieversorgungsaufnahme angeordnet. Dies erleichtert die Handhabung des chirurgischen Gleichstromelektrowerkzeugs, insbesondere den Austausch einer netzunabhängigen Energieversorgung durch ein chirurgisches Schaltnetzteil.

Um insbesondere ein unsteriles chirurgisches Schaltnetzteil in Verbindung mit dem chirurgischen Gleichstromelektrowerkzeug unter sterilen Bedingungen nutzen zu können, ist es günstig, wenn das Gleichstromelektrowerkzeug einen Gehäusedeckel zum Verschließen der Energieversorgungsaufnahme umfasst. Vorzugsweise ist der Gehäusedeckel relativ zum Gleichstromelektrowerkzeug fluiddicht abdichtbar.

Besonders einfach wird der Aufbau des chirurgischen Gleichstromwerkzeugs, wenn mindestens ein Teil des Schaltnetzteilgehäuses den Gehäusedeckel bildet. So ist es möglich, das chirurgische Schaltnetzteil mit dem Gleichstromelektrowerkzeug zu verbinden, wodurch die Energieversorgungsaufnahme durch das Schaltnetzteilgehäuse selbst verschlossen wird. Ein zusätzlicher Gehäusedeckel ist dann nicht erforderlich.

Vorzugsweise sind am Gehäusedeckel elektrische Anschlusskontakte vorgesehen, die auf einer Innenseite des Gehäusedeckels direkt oder indirekt mit dem chirurgischen Schaltnetzteil und auf einer Außenseite des Deckels mit einer Netzanschlussleitung verbindbar sind. Es lässt sich so eine Netzanschlussleitung auf einfache Weise vom chirurgischen Gleichstromelektrowerkzeug trennen und auch wieder mit diesem verbinden. Außerdem können so unterschiedliche Netzanschlussleitungen mit unterschiedlichen Steckadaptern für unterschiedliche Stromnetze auf einfache Weise mit dem chirurgischen Gleichstromelektrowerkzeug verbunden werden.

Günstig ist es, wenn eine Netzanschlussleitung vorgesehen ist zum lösbaren Verbinden des Gleichstromelektrowerkzeugs mit einer Wechselspannungsenergieversorgung.

Gemäß einer bevorzugten Ausführungsform der Erfindung kann vorgesehen sein, dass das chirurgische Schaltnetzteil eines der oben beschriebenen chirurgischen Schaltnetzteile ist.

Grundsätzlich wäre es denkbar, dass der Gleichstromelektroverbraucher in Form einer elektrischen Heizung, oder allgemein ausgedrückt, in Form eines Bauelements ohne bewegliche Teile ausgebildet ist. Vorzugsweise ist der Gleichstromelektroverbraucher ein Gleichstromelektromotor. Ein Gleichstromelektromotor kann insbesondere zur Ausbildung einer Bohr- oder Fräsmaschine oder einer Säge dienen.

Vorteilhaft ist es, wenn das chirurgische Gleichstromelektrowerkzeug eine chirurgische Bohrmaschine oder eine chirurgische Säge ist. Derartige Gleichstromelektrowerkzeuge können auf vielfältige Weise in Verbindung mit chirurgischen Eingriffen verwendet werden.

Die nachfolgende Beschreibung bevorzugter Ausführungsformen der Erfindung dient im Zusammenhang mit der Zeichnung der näheren Erläuterung. Es zeigen:
- Figur 1:: eine perspektivische schematische Darstellung eines chirurgischen Gleichstromelektrowerkzeugs mit einer netzunabhängigen Energieversorgung;
- Figur 2:: eine schematische Darstellung eines chirurgischen Gleichstromelektrowerkzeugs mit einem ersten Ausführungsbeispiel eines chirurgischen Schaltnetzteils;
- Figur 3:: eine schematische Darstellung eines chirurgischen Gleichstromelektrowerkzeugs mit einem zweiten Ausführungsbeispiel eines chirurgischen Schaltnetzteils;
- Figur 4:: eine schematische Darstellung eines chirurgischen Gleichstromelektrowerkzeugs mit einem dritten Ausführungsbeispiel eines chirurgischen Schaltnetzteils;
- Figur 5:: eine schematische Darstellung einer alternativen Ausführungsform eines Gehäusedeckels zum Verschließen einer Energieversorgungsaufnahme eines chirurgischen Gleichstromelektrowerkzeugs;
- Figur 6:: eine schematische Darstellung eines chirurgischen Gleichstromelektrowerkzeugs mit einem vierten Ausführungsbeispiel eines chirurgischen Schaltnetzteils;
- Figur 7:: eine schematische Darstellung eines chirurgischen Gleichstromelektrowerkzeugs mit einem fünften Ausführungsbeispiel eines chirurgischen Schaltnetzteils; und
- Figur 8:: eine schematische Darstellung einer alternativen Ausführungsform einer Schaltnetzteilschaltungsanordnung.

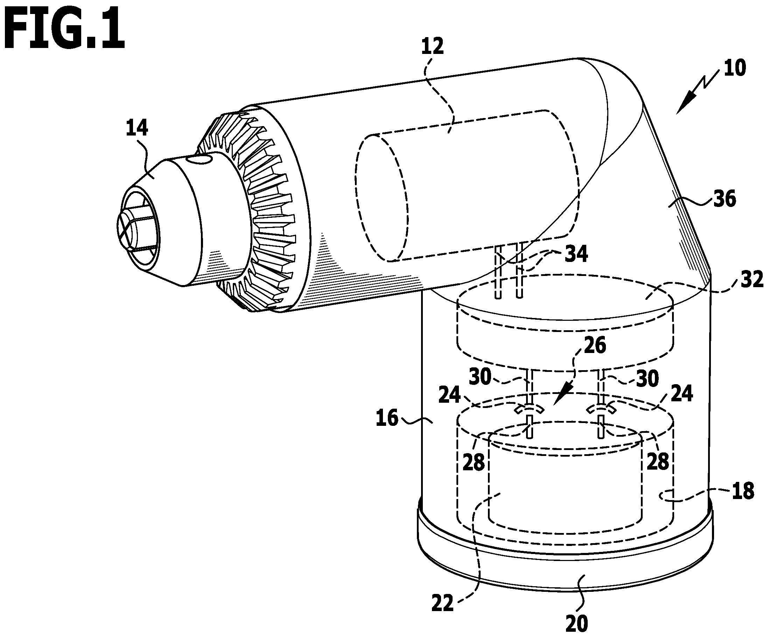

In Figur 1 ist schematisch ein insgesamt mit dem Bezugszeichen 10 versehenes Gleichstromelektrowerkzeug dargestellt, und zwar beispielhaft in Form einer chirurgischen Bohrmaschine. Diese umfasst einen Gleichstromelektroverbraucher 12 in Form eines Gleichstrommotors. Der Gleichstrommotor kann insbesondere in Form eines elektronisch kommutierten Gleichstrommotors ausgebildet sein. Der Gleichstromverbraucher 12 dient zum Antreiben einer nicht dargestellten Antriebswelle, die mit einer Kupplung 14 mechanisch gekoppelt ist, welche zur Verbindung mit chirurgischen Werkzeugen, beispielsweise Bohrern oder Fräsern, dient.

Ein Griffbereich 16 des Gleichstromelektrowerkzeugs 10 ist mit einer Energieversorgungsaufnahme 18 in Form einer Ausnehmung ausgestattet, welche mit einem Gehäusedeckel 20 verschließbar ist, und zwar vorzugsweise fluiddicht. Die Energieversorgungsaufnahme 18 ist derart ausgebildet, dass sie eine netzunabhängige Energiequelle 22, beispielsweise in Form einer Batterie oder eines wiederaufladbaren Akkumulators, aufnehmen kann. In der Energieversorgungsaufnahme 18 vorgesehene elektrische Kontakte 24 bilden eine elektrische Schnittstelle 26. Die Schnittstelle 26 ist derart ausgebildet und angeordnet, dass die elektrischen Kontakte 24 bei eingesetzter Energiequelle 22 mit Anschlusskontakten 28 derselben in Kontakt und/oder in Eingriff bringbar sind. Die Schnittstelle 26 ist über Leitungen 30 elektrisch mit einer in Figur 1 schematisch dargestellten Steuer- und/oder Regelungsschaltung 32 verbunden, die zur Steuer- und/oder Regelung des Gleichstromelektroverbrauchers 12 dient. Zu diesem Zweck ist die Steuer- und/oder Regelungsschaltung 32 über Leitungen 34 elektrisch mit dem Gleichstromelektroverbraucher 12 verbunden. Alternativ kann die Steuer- und/oder Regelungsschaltung 32 auch in die Energiequelle 22 integriert sein und mit dieser zusammen eine sogenannte Energie- und Steuerungseinheit bilden. In einem Gehäuse 36 des Gleichstromelektrowerkzeugs ist bei einer solchen Ausgestaltung dann lediglich der Gleichstromelektroverbraucher 12 angeordnet.

Für den Fall, dass kein netzunabhängiger Betrieb des Gleichstromelektrowerkzeugs 10 gewünscht ist, insbesondere in Fällen, in denen die Kapazität der Energiequelle 22 für einen chirurgischen Eingriff nicht vollständig ausreicht, kann letztere auch durch ein chirurgisches Schaltnetzteil 38 ersetzt werden.

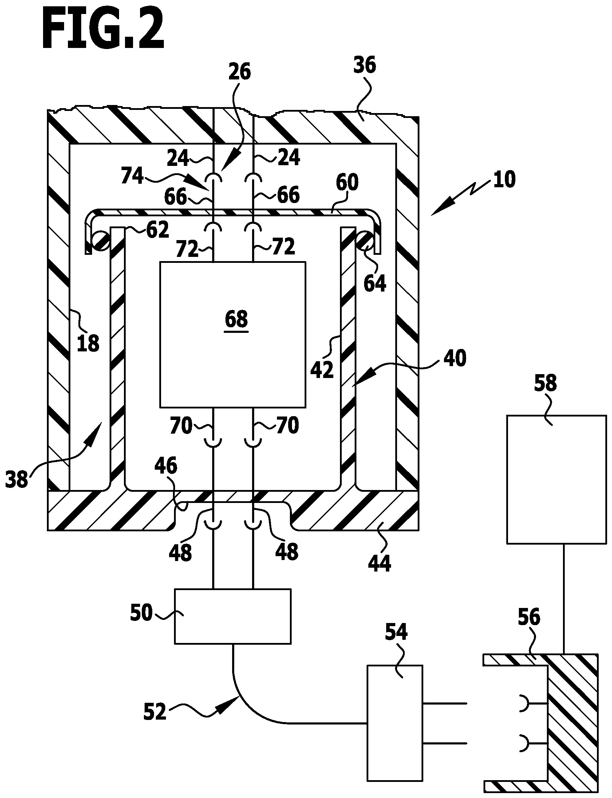

Ein erstes Ausführungsbeispiel eines Schaltnetzteils 38 ist in Figur 2 schematisch dargestellt. Es umfasst ein im Wesentlichen hülsenförmiges Schaltnetzteilgehäuse 40, welches eine im Wesentlichen hohlzylindrische Schaltungsaufnahme 42 definiert. Ein Boden 44 des Schaltnetzteilgehäuses 40 ist in radialer Richtung flanschartig erweitert und bildet so gleichzeitig einen Gehäusedeckel zum Verschließen der Energieversorgungsaufnahme 18. An einer Außenseite des Bodens 44 ist eine Aussparung 46 mit Anschlusskontakten 48 vorgesehen, die eine Kaltgerätesteckdose zusammen bilden und mit einem Steckverbinder 50 eines Netzanschlusskabels 52 verbindbar sind, welches wiederum einen Netzstecker 54 an seinem anderen Ende aufweist, welches mit einer an ein Stromnetz 58 angeschlossenen Steckdose 56 verbindbar ist.

Das Schaltnetzteilgehäuse 40 umfasst ferner einen Schaltnetzteilgehäusedeckel 60 zum Verschließen einer Schaltnetzteilgehäuseöffnung 62. Eine Dichtung 64 ist die Schaltnetzteilgehäuseöffnung 62 umgebend am Schaltnetzteilgehäuse 40 beziehungsweise am Schaltnetzteilgehäusedeckel 60 angeordnet, um die Schaltungsaufnahme 42 keimdicht abzudichten. Den Deckel durchsetzen erste elektrische Kontakte 66, die eine Schaltnetzteilschnittstelle bilden, die mit der Schnittstelle 26 des Gleichstromelektrowerkzeugs 10 in Kontakt und/oder Eingriff bringbar und/oder verbindbar ist. Die ersten elektrischen Kontakte 66 durchsetzen den Schaltnetzteilgehäusedeckel 60. Die Anschlusskontakte 48 durchsetzen den Boden 44 des Schaltnetzteilgehäuses 40.

In die Schaltungsaufnahme 42 einsetzbar ist eine Schaltnetzteilschaltungsanordnung 68, die insbesondere eine Gleichrichterschaltung zum Wandeln einer Wechselspannung in eine Gleichspannung umfasst. Ferner umfasst sie Netzanschlusskontakte 70, die mit den Anschlusskontakten 48 verbindbar sind zum direkten oder indirekten Verbinden mit einer Wechselspannungsenergieversorgung, beispielsweise dem Stromnetz 58. Ferner sind an der Schaltnetzteilschaltungsanordnung 68 Gleichstromanschlusskontakte 72 vorgesehen, die mit den ersten elektrischen Kontakten 66 verbindbar sind zum indirekten Verbinden mit der Schnittstelle 26 des Gleichstromelektrowerkzeugs 10.

Das Schaltnetzteil 38 ermöglicht es, die Schaltnetzteilschaltungsanordnung 68 über eine nicht dargestellte Einführhilfe, beispielsweise in Form eines Steriltrichters, in das zuvor sterilisierte Schaltnetzteilgehäuse 40 einzuführen. Anschließend wird das Schaltnetzteilgehäuse 40 mit dem Schaltnetzteilgehäusedeckel 60 keimdicht verschlossen. Das Schaltnetzteil 38 kann dann in die Energieversorgungsaufnahme 18 des Gleichstromelektrowerkzeugs 10 eingeführt werden, so dass die Schnittstelle 26 mit einer Schaltnetzschnittstelle 74 des Schaltnetzteils 38, die insbesondere die ersten elektrischen Kontakte 66 umfasst, in Kontakt und/oder in Eingriff gebracht werden kann. Optional kann insbesondere das Schaltnetzteilgehäuse 40 auch mechanisch mit dem Gehäuse 36 verbunden werden, beispielsweise durch Verrasten oder Verschrauben.

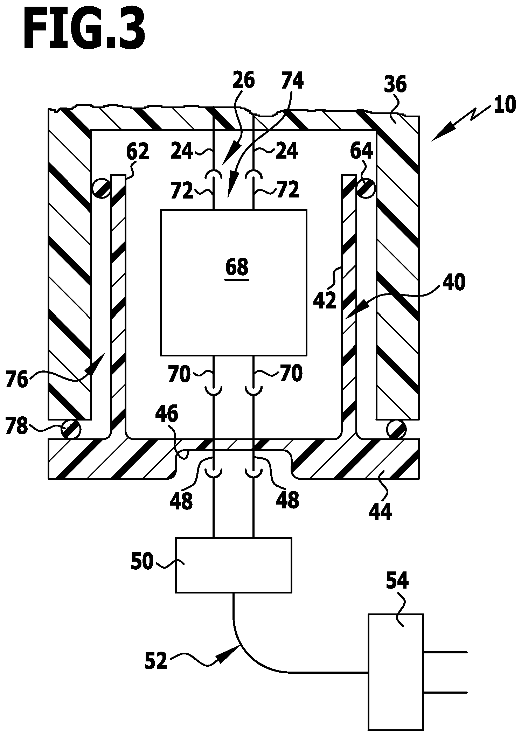

Ein zweites Ausführungsbeispiel eines chirurgischen Schaltnetzteils ist in Figur 3 schematisch dargestellt und insgesamt mit dem Bezugszeichen 76 versehen. Das Schaltnetzteil 76 unterscheidet sich vom Schaltnetzteil 38 nur dadurch, dass das Schaltnetzteilgehäuse 40 keinen Schaltnetzteilgehäusedeckel 60 umfasst. Die Gleichstromanschlusskontakte 72 der Schaltnetzteilschaltungsanordnung 68 bilden daher selbst eine Schaltnetzteilschnittstelle 74, die mit der Schnittstelle 26 in Eingriff bringbar ist. Die Dichtung 64 ist die Schaltnetzteilgehäuseöffnung 62 umgebend am Schaltnetzteilgehäuse 40 angeordnet. Sie dient zur Abdichtung der Schaltungsaufnahme 42 relativ zum Gehäuse 36. Optional kann eine weitere Dichtung 78 vorgesehen sein, die die vom Boden 44 gebildete flanschartige Verbreiterung zusätzlich relativ zum Gehäuse 36 des Gleichstromelektrowerkzeugs 10 abdichtet. Die Dichtung 78 kann entweder am Boden 44 oder am Gehäuse 36 gehalten sein.

Auch das Schaltnetzteil 76 ermöglicht es, eine unsterile Schaltnetzteilschaltungsanordnung 68 in das zuvor sterilisierte Schaltnetzteilgehäuse 40 einzusetzen, beispielsweise über den oben erwähnten Steriltrichter. Anschließend wird das Schaltnetzteil 76 in die Energieversorgungsaufnahme 18 eingeführt und mittels der Dichtung 64 beziehungsweise optional auch mit der Dichtung 78 relativ zum Gehäuse 36 keimdicht abgedichtet.

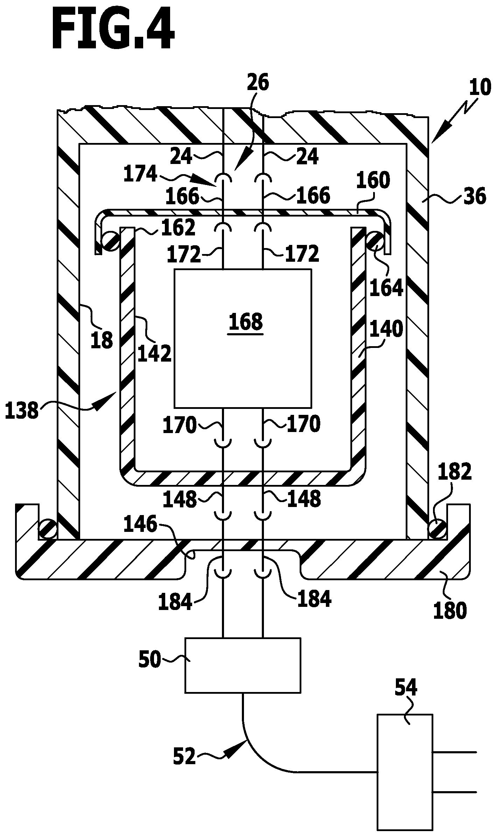

Ein drittes Ausführungsbeispiel eines Schaltnetzteils ist schematisch in Figur 4 dargestellt und insgesamt mit dem Bezugszeichen 138 versehen. Es weist große Ähnlichkeit mit dem Schaltnetzteil 38 auf, so dass Teile des Schaltnetzteils 138, die Teilen des Schaltnetzteils 38 entsprechen, mit Bezugszeichen versehen sind, die dieselben beiden Endziffern aufweisen. Der wesentliche Unterschied zu dem im Zusammenhang mit Figur 2 beschriebenen Schaltnetzteil 38 besteht darin, dass ein separater Gehäusedeckel 180 vorgesehen ist, mit dem die Energieversorgungsaufnahme 18 verschließbar ist. Eine fluiddichte/ keimdichte Abdichtung wird durch eine Dichtung 182 erreicht, die zwischen dem Gehäusedeckel 180 und dem Gehäuse 36 angeordnet und entweder am Gehäusedeckel 180 oder am Gehäuse 36 gehalten ist. Am Gehäusedeckel 180 ist eine Kaltgerätesteckdose ausgebildet durch Vorsehen einer nach außen weisenden Aussparung 146 an der vierte elektrische Kontakte 184 angeordnet sind, die zudem den Gehäusedeckel 180 durchsetzen und mit den aus dem Schaltnetzteilgehäuse 140 vorstehenden Anschlusskontakten 148 elektrisch verbindbar sind. Mit der Kaltgerätesteckdose des Gehäusedeckels 180 ist wiederum in der oben beschriebenen Weise das Netzanschlusskabel 52 verbindbar.

Einen separaten Gehäusedeckel 180 vorzusehen, hat insbesondere den Vorteil, dass nicht nur eine unsterile Schaltnetzteilschaltungsanordnung 168 verwendet werden kann, sondern auch ein unsteriles Schaltnetzteilgehäuse 140. Die Schaltnetzteilschaltungsanordnung 168 kann folglich zunächst in die Schaltungsaufnahme 142 eingesetzt werden. Anschließend kann die Schaltnetzteilgehäuseöffnung 162 mit einem Schaltnetzteilgehäusedeckel 160 verschlossen werden. Auf den Schaltnetzteilgehäusedeckel 160 kann optional jedoch auch verzichtet werden und die Gleichstromanschlusskontakte statt mit den ersten elektrischen Kontakten 166 direkt mit der Schnittstelle 26 verbunden werden. Das so vorbereitete Schaltnetzteil 138 kann dann zum Beispiel mittels einer oben beschriebenen Einführhilfe in die Energieversorgungsaufnahme 18 eingeführt und diese dann mit dem Gehäusedeckel 180 keimdicht verschlossen werden.

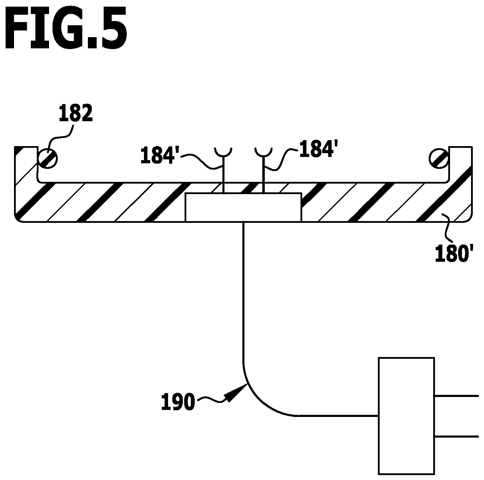

Anstelle einer Kaltgerätesteckdose kann an einem Gehäusedeckel 180' ein Netzanschlusskabel 190 dauerhaft angeschlossen sein. Es kann so insbesondere auf einen Steckverbinder am Anschlusskabel 190 zur Verbindung mit der nicht vorhandenen Kaltgerätesteckdose des Gehäusedeckels 190 verzichtet werden. Das Netzanschlusskabel 190 wird vielmehr direkt mit den vierten elektrischen Kontakten 184' verbunden, die wiederum mit den Anschlusskontakten 148 beispielsweise des Schaltnetzteils 138 verbindbar sind. Eine schematische Darstellung des Gehäusedeckels 180' ist in Figur 5 gezeigt.

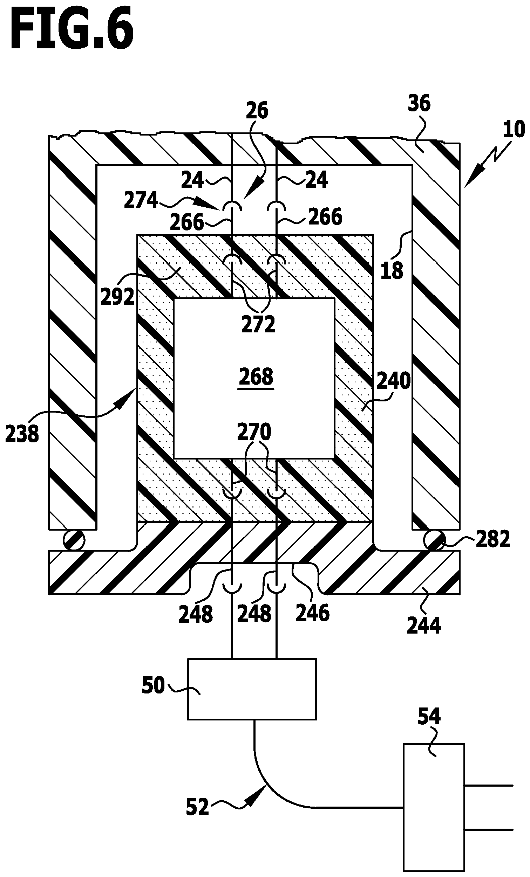

Ein viertes Ausführungsbeispiel eines chirurgischen Schaltnetzteils ist in Figur 6 schematisch dargestellt und insgesamt mit dem Bezugszeichen 238 versehen. Es entspricht in seinem Aufbau im Wesentlichen dem Schaltnetzteil 38, jedoch in der in Figur 3 dargestellten Variante ohne Schaltnetzteilgehäusedeckel 60. Die Schaltnetzteilschaltungsanordnung 268 ist, beispielsweise durch Vakuumverguss mit einem Epoxidharz 292, fest in das Schaltnetzteilgehäuse 240 eingebaut. Auf diese Weise sind insbesondere feuchtigkeits- und temperaturempfindliche elektronische Bauelemente der Schaltnetzteilschaltungsanordnung 268 geschützt, so dass das eine einzige Einheit bildende Schaltnetzteil 238 selbst auch sterilisierbar ist. Optional kann auch der Verguss das Schaltnetzteilgehäuse bilden. Eine am Boden 244 des Schaltnetzteilgehäuses 238 vorgesehene Kaltgerätesteckdose kann wiederum mit einem Netzanschlusskabel 52 verbunden werden.

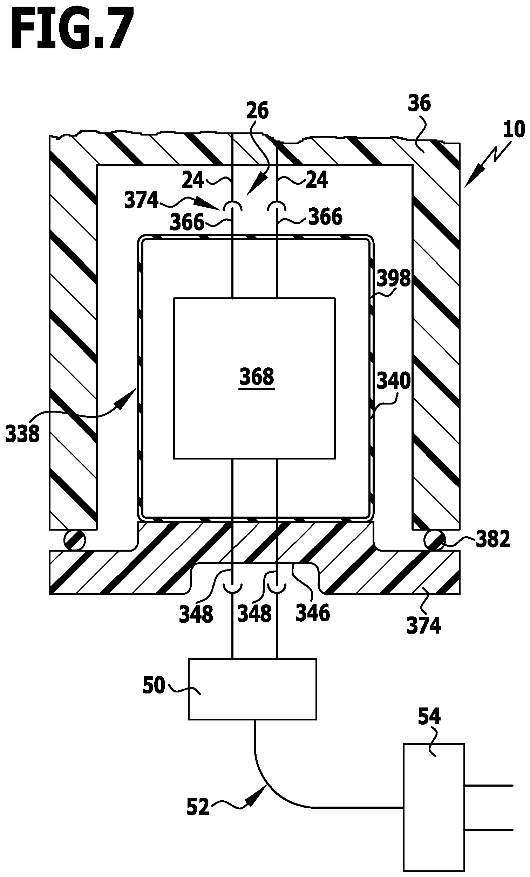

Bei einem schematisch in Figur 7 dargestellten fünften Ausführungsbeispiel eines Schaltnetzteils 338 wird eine vollständige Sterilisierbarkeit desselben dadurch erreicht, dass die Schaltnetzteilschaltungsanordnung 368 vollständig in eine feuchtigkeitsabweisende Membran 300 eingehüllt ist. Die Membran 300 kann beispielsweise als innere Beschichtung des Gehäuses 340 vorgesehen sein. Im Übrigen entspricht der Aufbau des Schaltnetzteils 338 dem Schaltnetzteil 238.

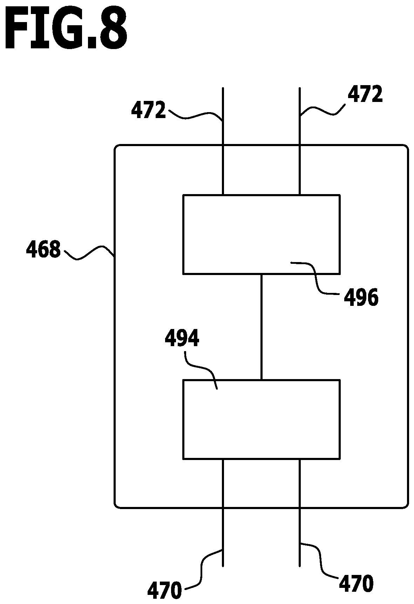

Falls das Gleichstromelektrowerkzeug 10, wie im Zusammenhang mit der Beschreibung von Figur 1 angedeutet, keine Steuer- und/oder Regelungsschaltung 32 für den Gleichstromelektroverbraucher 12 aufweist, muss eine solche Steuer- und/oder Regelungsschaltung 496 entweder an der netzunabhängigen Energiequelle 22 oder an einem Schaltnetzteil vorgesehen werden. In Figur 8 ist schematisch eine Schaltnetzteilschaltungsanordnung 468 dargestellt, die sowohl eine Wechselrichterschaltung 494 als auch eine Steuer- und/oder Regelungsschaltung 496 zur Steuerung und/oder Regelung des Gleichstromelektroverbrauchers 12 des Gleichstromelektrowerkzeugs 10 aufweist. Ferner weist die Schaltnetzteilschaltungsanordnung 468 Netzanschlusskontakte 470 zum direkten oder indirekten Verbinden mit einer Wechselspannungsenergieversorgung und Gleichstromanschlusskontakte 472 zum direkten oder indirekten Verbinden mit dem Gleichstromverbraucher 12 des Gleichstromelektrowerkzeugs 10 auf.

Die Schaltnetzteilschaltungsanordnung 468 kann optional die Schaltnetzteilschaltungsanordnungen 68, 168, 268 und 368 ersetzen.

Auch bei den im Zusammenhang mit den Figuren 2, 3, 4, 6 und 7 beschriebenen Netzanschlussleitungen 52 können alternativ, wie im Zusammenhang mit Figur 5 beschrieben, Netzanschlussleitungen unlösbar mit einem Gehäusedeckel des Gehäuses 36 oder mit dem Schaltnetzteilgehäuse des jeweiligen Schaltnetzteils verbunden sein.

## Patentansprüche

1. Chirurgisches Gleichstromelektrowerkzeug (10) mit einem Gehäuse (36), einem im Gehäuse (36) aufgenommenen Gleichstromelektroverbraucher (12) und einer Schnittstelle (26) für eine netzunabhängige Energiequelle (22), wobei das Gehäuse (36) eine Energieversorgungsaufnahme (18) für die netzunabhängige Energiequelle (22) aufweist, **gekennzeichnet durch** ein chirurgisches Schaltnetzteil (38; 76; 138; 238; 338), welches eine Schaltnetzteilschaltungsanordnung (68; 168; 268; 368; 468), ein Schaltnetzteilgehäuse (40; 140; 240; 340) und eine im Schaltnetzteilgehäuse (40; 140; 240; 340) ausgebildete Schaltungsaufnahme (42; 142; 242; 342) zum Aufnehmen der Schaltnetzteilschaltungsanordnung (68; 168; 268; 368; 468) umfasst, und dass das Schaltnetzteilgehäuse (40; 140; 240; 340) eine Schaltnetzteilschnittstelle (74; 174; 274; 374) aufweist, die derart ausgebildet ist, dass sie mit der für die netzunabhängige Energiequelle (22) vorgesehenen Schnittstelle (26) des Gleichstromelektrowerkzeugs (10) in Eingriff bringbar ist, dass das chirurgische Schaltnetzteil (38; 76; 138; 238; 338) derart ausgebildet ist, dass es in die Energieversorgungsaufnahme (18) vollständig einführbar und in dieser festlegbar ist, dass mindestens eine Dichtung (64, 78; 164, 182; 282; 382) vorgesehen ist zum fluiddichten Abdichten der Schaltungsaufnahme (42; 142; 242; 342) und dass die mindestens eine Dichtung (64; 78; 182; 282; 382) derart angeordnet ist, dass sie das Schaltnetzteilgehäuse (40; 140; 240; 340) relativ zum Gehäuse (36) oder der Energieversorgungsaufnahme (18) des Gehäuses (10) abdichtet.

2. Chirurgisches Gleichstromelektrowerkzeug nach Anspruch 1, **dadurch gekennzeichnet, dass**
a) die Schaltnetzteilschnittstelle (74; 174; 274; 374) derart ausgebildet ist, dass sie mit der Schnittstelle (26) des Gleichstromelektrowerkzeugs (10) lösbar verbindbar ist,
und/oder
b) die Schaltnetzteilschnittstelle (74; 174; 274; 374) derart ausgebildet ist, dass sie mit der Schnittstelle (26) des Gleichstromelektrowerkzeugs (10) elektrisch und/oder mechanisch verbindbar ist,
und/oder
c) die Schnittstelle (26) an oder in der Energieversorgungsaufnahme (18) angeordnet ist.

3. Chirurgisches Gleichstromelektrowerkzeug nach Anspruch 1 oder 2, **dadurch gekennzeichnet, dass** das Gleichstromelektrowerkzeug (10) einen Gehäusedeckel (40; 180; 180'; 240; 340) zum Verschließen der Energieversorgungsaufnahme (18) umfasst.

4. Chirurgisches Gleichstromelektrowerkzeug nach Anspruch 3, **dadurch gekennzeichnet, dass**
a) mindestens ein Teil des Schaltnetzteilgehäuses (40; 240; 340) den Gehäusedeckel bildet
und/oder
b) am Gehäusedeckel (40; 180; 180'; 240; 340) elektrische Anschlusskontakte (48; 184; 184'; 248; 348) vorgesehen sind, die auf einer Innenseite des Gehäusedeckels (40; 180; 180'; 240; 340) direkt oder indirekt mit dem chirurgischen Schaltnetzteil (38; 76; 138; 238; 338) und auf einer Außenseite des Deckels (40; 180; 180'; 240; 340) mit einer Netzanschlussleitung (52; 190) verbindbar sind.

5. Chirurgisches Gleichstromelektrowerkzeug nach einem der voranstehenden Ansprüche, **dadurch gekennzeichnet, dass**
a) eine Netzanschlussleitung (52; 190) vorgesehen ist zum lösbaren Verbinden des Gleichstromelektrowerkzeugs (10) mit einer Wechselspannungsenergieversorgung (58)
und/oder
b) der Gleichstromelektroverbraucher (12) ein Gleichstromelektromotor ist
und/oder
c) das chirurgische Gleichstromelektrowerkzeug (10) eine chirurgische Bohrmaschine oder eine chirurgische Säge ist.

6. Chirurgisches Gleichstromelektrowerkzeug nach einem der voranstehenden Ansprüche, **dadurch gekennzeichnet, dass** die Schaltnetzteilschaltungsanordnung (368) von einer feuchtigkeitsabweisenden Membran (398) umgeben ist.

7. Chirurgisches Gleichstromelektrowerkzeug nach einem der voranstehenden Ansprüche, **dadurch gekennzeichnet, dass** eine Gleichstromverbraucher-Steuer- und/oder Regelungsschaltung (496) vorgesehen ist, die mindestens teilweise vom Schaltnetzteilgehäuse (40; 140; 240; 340) umgeben ist,
wobei insbesondere die Schaltnetzteilschaltungsanordnung (468) die Gleichstromverbraucher-Steuer- und/oder Regelungsschaltung (496) umfasst.

8. Chirurgisches Gleichstromelektrowerkzeug nach einem der voranstehenden Ansprüche, **dadurch gekennzeichnet, dass** eine Einführhilfe vorgesehen ist zum Einführen der Schaltnetzteilschaltungsanordnung (68; 168) in das Schaltnetzteilgehäuse (40; 140),
wobei insbesondere die Einführhilfe trichterförmig ausgebildet ist.

9. Chirurgisches Gleichstromelektrowerkzeug nach einem der voranstehenden Ansprüche, **dadurch gekennzeichnet, dass** die Schaltnetzteilschaltungsanordnung (68; 168; 268; 368; 468)
a) eine Gleichrichterschaltung (494) zum Wandeln einer Wechselspannung in eine Gleichspannung umfasst
und/oder
b) Netzanschlusskontakte (70; 170; 270; 370; 470) zum direkten oder indirekten Verbinden mit einer Wechselspannungsenergieversorgung (58) und Gleichstromanschlusskontakte (72; 172; 272; 372; 472) zum direkten oder indirekten Verbinden mit dem Gleichstromelektrowerkzeug (10) oder dem Gleichstromverbraucher (12) desselben aufweist.

10. Chirurgisches Gleichstromelektrowerkzeug nach einem der voranstehenden Ansprüche, **dadurch gekennzeichnet, dass** das Schaltnetzteilgehäuse (40; 140)
a) eine Schaltnetzteilgehäuseöffnung (62; 162) aufweist, durch die die Schaltnetzteilschaltungsanordnung (68; 168) in die Schaltungsaufnahme (42; 142) einführbar ist, und dass das Schaltnetzteilgehäuse (40; 140) einen Schaltnetzteilgehäusedeckel (60; 160) zum Verschließen der Schaltnetzteilgehäuseöffnung (62; 162) umfasst
und/oder
b) dampfsterilisierbar ist
und/oder
c) aus mindestens einem Kunststoff hergestellt ist.

11. Chirurgisches Gleichstromelektrowerkzeug nach einem der voranstehenden Ansprüche, **dadurch gekennzeichnet, dass** die mindestens eine Dichtung (64; 164)
a) die Schaltnetzteilgehäuseöffnung (62; 162) umgebend am Schaltnetzteilgehäuse (40; 140) angeordnet ist
und/oder
b) derart angeordnet ist, dass sie das Schaltnetzteilgehäuse (40; 140) und den Schaltnetzteilgehäusedeckel (60; 160) relativ zueinander abdichtet, wenn der Schaltnetzteilgehäusedeckel (60; 160) die Schaltnetzteilgehäuseöffnung (62; 162) verschließt.

12. Chirurgisches Gleichstromelektrowerkzeug nach einem der voranstehenden Ansprüche, **dadurch gekennzeichnet, dass** am Schaltnetzteilgehäuse (40; 140; 240; 340) erste elektrische Kontakte (66; 166; 266; 366) vorgesehen sind zur Verbindung der Schaltnetzteilschaltungsanordnung (68; 168; 268; 368) mit dem Gleichstromelektroverbraucher (12).

13. Chirurgisches Gleichstromelektrowerkzeug nach Anspruch 12, **dadurch gekennzeichnet, dass** die ersten elektrischen Kontakte (66; 166) am Schaltnetzteilgehäusedeckel (60; 160) angeordnet sind.

14. Chirurgisches Gleichstromelektrowerkzeug nach einem der voranstehenden Ansprüche, **dadurch gekennzeichnet, dass** am Schaltnetzteilgehäuse (40; 140; 240; 340) zweite elektrische Kontakte (48; 148; 248; 348) vorgesehen sind zur Verbindung mit dritten elektrischen Kontakten einer Netzanschlussleitung (52; 190) oder zur Verbindung mit vierten elektrischen Kontakten (184; 184') eines Gehäusedeckels (180; 180') des Gleichstromelektrowerkzeugs (10).

15. Chirurgisches Gleichstromelektrowerkzeug nach einem der voranstehenden Ansprüche, **dadurch gekennzeichnet, dass** die Schaltnetzteilschaltungsanordnung (268; 368)
a) sterilisierbar ist
und/oder
b) in einen sterilisierbaren Kunststoff (392) eingegossen ist, wobei insbesondere der Kunststoff (392) ein Epoxidharz ist,
und/oder
c) die Schaltnetzteilschaltungsanordnung (268; 368) und das Schaltnetzteilgehäuse (240; 340) unlösbar miteinander verbunden sind.

## Claims

1. Surgical DC power tool (10) with a housing (36), a DC power consumer (12) accommodated in the housing (36) and an interface (26) for a mains-independent energy source (22), wherein the housing (36) has an energy supply receptacle (18) for the mains-independent energy source (22), **characterized by** a surgical switch mode power supply (38; 76; 138; 238; 338), said power supply comprising a switch mode power supply circuit arrangement (68; 168; 268; 368; 468), a switch mode power supply housing (40; 140; 240; 340) and a circuit receptacle (42; 142; 242; 342) formed in the switch mode power supply housing (40; 140; 240; 340) for accommodating the switch mode power supply circuit arrangement (68; 168; 268; 368; 468), and in that the switch mode power supply housing (40; 140; 240; 340) has a switch mode power supply interface (74; 174; 274; 374) designed in such a manner that it is adapted to be brought into engagement with the interface (26) of the DC power tool (10) provided for the mains-independent energy source (22), in that the surgical switch mode power supply (38; 76; 138; 238; 338) is designed in such a manner that it is adapted to be inserted completely into the energy supply receptacle (18) and fixed in it, in that at least one seal (64, 78; 164, 182: 282; 382) is provided for the fluid-tight sealing of the circuit receptacle (42; 142; 242; 342) and in that the at least one seal (64; 78; 182; 282; 382) is arranged in such a manner that it seals the switch mode power supply housing (40; 140; 240; 340) relative to the housing (36) or the energy supply receptacle (18) of the housing (10).

2. Surgical DC power tool according to claim 1, **characterized in that**
a) the interface (74; 174; 274; 374) of the switch mode power supply is designed in such a manner that it is detachably connectable to the interface (26) of the DC power tool (10),
and/or
b) the interface (74; 174; 274; 374) of the switch mode power supply is designed in such a manner that it is connectable to the interface (26) of the DC power tool (10) electrically and/or mechanically,
and/or
c) the interface (26) is arranged at or in the energy supply receptacle (18).

3. Surgical DC power tool according to claim 1 or 2, **characterized in that** the DC power tool (10) comprises a housing cover (40; 180; 180'; 240; 340) for closing the energy supply receptacle (18).

4. Surgical DC power tool according to claim 3, **characterized in that**
a) at least one part of the housing (40; 240; 340) of the switch mode power supply forms the housing cover
and/or
b) electrical connection contacts (48; 184; 184'; 248; 348) are provided on the housing cover (40; 180; 180'; 240; 340), said contacts being connectable directly or indirectly to the surgical switch mode power supply (38; 76; 138; 238; 338) on an inner side of the housing cover (40; 180; 180'; 240; 340) and to a mains connection line (52; 190) on an outer side of the cover (40; 180; 180'; 240; 340).

5. Surgical DC power tool according to any one of the preceding claims, **characterized in that**
a) a mains connection line (52; 190) is provided for the detachable connection of the DC power tool (10) to an AC voltage energy supply (58)
and/or
b) the DC power consumer (12) is a DC electric motor
and/or
c) the surgical DC power tool (10) is a surgical drill or a surgical saw.

6. Surgical switch mode power supply according to any one of the preceding claims, **characterized in that** the switch mode power supply circuit arrangement (368) is surrounded by a moisture-repellant membrane (398).

7. Surgical switch mode power supply according to any one of the preceding claims, **characterized in that** a DC consumer control and/or regulating circuit (496) is provided, said circuit being surrounded at least partially by the switch mode power supply housing (40; 140; 240; 340), wherein, in particular, the switch mode power supply circuit arrangement (468) comprises the DC consumer control and/or regulating circuit (496).

8. Surgical switch mode power supply according to any one of the preceding claims, **characterized in that** an insertion aid is provided for the insertion of the circuit arrangement (68; 168) of the switch mode power supply into the switch mode power supply housing (40; 140), wherein, in particular, the insertion aid is of a funnel-shaped design.

9. Surgical switch mode power supply according to any one of the preceding claims, **characterized in that** the circuit arrangement (68; 168; 268; 368; 468) of the switch mode power supply
a) comprises a rectifier circuit (494) for converting an AC voltage into a DC voltage
and/or
b) has mains connection contacts (70; 170; 270; 370; 470) for the direct or indirect connection to an AC voltage energy supply (58) and DC connection contacts (72; 172; 272; 372; 472) for the direct or indirect connection to the DC power tool (10) or the DC consumer (12) thereof.

10. Surgical switch mode power supply according to any one of the preceding claims, **characterized in that** the switch mode power supply housing (40; 140)
a) has a switch mode power supply housing opening (62; 162), the switch mode power supply circuit arrangement (68; 168) being insertable through said opening into the circuit receptacle (42; 142), and **in that** the switch mode power supply housing (40; 140) comprises a switch mode power supply housing cover (60; 160) for closing the switch mode power supply housing opening (62; 162)
and/or
b) is adapted to be sterilized by steam
and/or
c) is produced from at least one plastic material.

11. Surgical switch mode power supply according to any one of the preceding claims, **characterized in that** the at least one seal (64; 164)
a) is arranged on the switch mode power supply housing (40; 140) so as to surround the switch mode power supply housing opening (62; 162)
and/or
b) is arranged in such a manner that it seals the switch mode power supply housing (40; 140) and the switch mode power supply housing cover (60; 160) relative to one another when the switch mode power supply housing cover (60; 160) closes the switch mode power supply housing opening (62; 162).

12. Surgical switch mode power supply according to any one of the preceding claims, **characterized in that** first electrical contacts (66; 166; 266; 366) are provided on the switch mode power supply housing (40; 140; 240; 340) for connecting the switch mode power supply circuit arrangement (68; 168; 268; 368) to the DC power consumer (12).

13. Surgical switch mode power supply according to claim 12, **characterized in that** the first electrical contacts (66; 166) are arranged on the switch mode power supply housing cover (60; 160).

14. Surgical switch mode power supply according to any one of the preceding claims, **characterized in that** second electrical contacts (48; 148; 248; 348) are provided on the switch mode power supply housing (40; 140; 240; 340) for the connection to third electrical contacts of a mains connection line (52; 190) or for the connection to fourth electrical contacts (184; 184') of a housing cover (180; 180') of the surgical DC power tool (10).

15. Surgical switch mode power supply according to any one of the preceding claims, **characterized in that** the switch mode power supply circuit arrangement (268; 368)
a) is adapted to be sterilized
and/or
b) is cast into a sterilizable plastic material (392),
wherein, in particular, the plastic material (392) is an epoxy resin,
and/or
c) the switch mode power supply circuit arrangement (268; 368) and the switch mode power supply housing (240; 340) are connected non-detachably to one another.

## Revendications

1. Outil chirurgical électrique à courant continu (10) avec un boîtier (36), un dissipateur électrique à courant continu (12) logé dans le boîtier (36) et une interface (26) pour une source d'énergie indépendante du réseau (22), où le boîtier (36) présente un logement d'alimentation en énergie (18) pour la source d'énergie indépendante du réseau (22), **caractérisé par** une alimentation à découpage chirurgicale (38 ; 76 ; 138 ; 238 ; 338), qui comprend un agencement de circuit d'alimentation à découpage (68 ; 168 ; 268 ; 368 ; 468), un boîtier d'alimentation à découpage (40 ; 140 ; 240 ; 340) et un logement de circuit (42 ; 142 ; 242 ; 342) formé dans le boîtier d'alimentation à découpage (40 ; 140 ; 240 ; 340) pour loger l'agencement de circuit d'alimentation à découpage (68 ; 168 ; 268 ; 368 ; 468), et en ce que le boîtier d'alimentation à découpage (40 ; 140 ; 240 ; 340) présente une interface d'alimentation à découpage (74 ; 174 ; 274 ; 374) qui est formée de telle manière qu'elle peut être amenée en prise avec l'interface (26) de l'outil électrique à courant continu (10) prévue pour la source d'énergie indépendante du réseau (22), en ce que l'alimentation à découpage chirurgicale (38 ; 76 ; 138 ; 238 ; 338) est formée de telle manière qu'elle peut être introduite totalement dans le logement d'alimentation en énergie (18) et être fixée dans celui-ci, en ce qu'au moins un joint d'étanchéité (64, 78 ; 164, 182 ; 282 ; 382) est prévu pour l'étanchéification étanche aux fluides du logement de circuit (42 ; 142 ; 242 ; 342) et en ce que le au moins un joint d'étanchéité (64, 78 ; 182 ; 282 ; 382) est agencé de telle manière qu'il étanchéifie le boîtier d'alimentation à découpage (40 ; 140 ; 240 ; 340) par rapport au boîtier (36) ou au logement d'alimentation en énergie (18) du boîtier (10).

2. Outil chirurgical électrique à courant continu selon la revendication 1, **caractérisé en ce que**
a) l'interface d'alimentation à découpage (74 ; 174 ; 274 ; 374) est formée de telle manière qu'elle peut être reliée de manière amovible avec l'interface (26) de l'outil électrique à courant continu (10),
et/ou
b) l'interface d'alimentation à découpage (74 ; 174 ; 274 ; 374) est formée de telle manière qu'elle peut être reliée de manière électrique et/ou mécanique avec l'interface (26) de l'outil électrique à courant continu (10),
et/ou
c) l'interface (26) est disposée sur ou dans le logement d'alimentation en énergie (18).

3. Outil chirurgical électrique à courant continu selon la revendication 1 ou 2, **caractérisé en ce que** l'outil électrique à courant continu (10) comprend un couvercle de boîtier (40 ; 180 ; 180' ; 240 ; 340) pour la fermeture du logement d'alimentation en énergie (18).

4. Outil chirurgical électrique à courant continu selon la revendication 3, **caractérisé en ce que**
a) au moins une partie du boîtier d'alimentation à découpage (40 ; 240 ; 340) forme le couvercle de boîtier
et/ou
b) il est prévu sur le couvercle de boîtier (40 ; 180 ; 180' ; 240 ; 340) des contacts de raccordement électriques (48 ; 184 ; 184' ; 248 ; 348) qui peuvent être reliés sur un côté intérieur du couvercle de boîtier (40 ; 180 ; 180' ; 240 ; 340) directement ou indirectement avec l'alimentation à découpage chirurgicale (38 ; 76 ; 138 ; 238 ; 338) et sur un côté extérieur du couvercle (40 ; 180 ; 180' ; 240 ; 340) avec un conducteur de raccordement au réseau (52 ; 190).

5. Outil chirurgical électrique à courant continu selon l'une des revendications précédentes, **caractérisé en ce que**
a) un conducteur de raccordement au réseau (52 ; 190) est prévu pour la liaison amovible de l'outil électrique à courant continu (10) avec une alimentation en énergie en courant alternatif (58)
et/ou
b) le dissipateur électrique en courant continu (12) est un moteur électrique à courant continu
et/ou
c) l'outil chirurgical électrique à courant continu (10) est une perceuse chirurgicale ou une scie chirurgicale.

6. Outil chirurgical électrique à courant continu selon l'une des revendications précédentes, **caractérisé en ce que** l'agencement de circuit d'alimentation à découpage (368) est entouré par une membrane repoussant l'humidité (398).

7. Outil chirurgical électrique à courant continu selon l'une des revendications précédentes, **caractérisé en ce qu'**il est prévu un circuit de commande et/ou de régulation de dissipateur à courant continu (496) qui est entouré au moins en partie par le boîtier d'alimentation à découpage (40 ; 140 ; 240 ; 340),
où en particulier l'agencement de circuit d'alimentation à découpage (468) comprend le circuit de commande et/ou de régulation de dissipateur à courant continu (496).

8. Outil chirurgical électrique à courant continu selon l'une des revendications précédentes, **caractérisé en ce qu'**il est prévu une aide d'introduction pour l'introduction de l'agencement de circuit d'alimentation à découpage (68 ; 168) dans le boîtier d'alimentation à découpage (40 ; 140),
où en particulier l'aide d'introduction est sous forme d'entonnoir.

9. Outil chirurgical électrique à courant continu selon l'une des revendications précédentes, **caractérisé en ce que** l'agencement de circuit d'alimentation à découpage (68 ; 168 ; 268 ; 368 ; 468)
a) comprend un circuit redresseur (494) pour convertir une tension alternative en une tension continue
et/ou
b) présente des contacts de raccordement au réseau (70 ; 170 ; 270 ; 370 ; 470) pour la liaison directe ou indirecte avec une alimentation en énergie en tension alternative (58) et des contacts de raccordement en courant continu (72 ; 172 ; 272 ; 372 ; 472) pour la liaison directe ou indirecte avec l'outil électrique à courant continu (10) ou le dissipateur à courant continu (12) de celui-ci.

10. Outil chirurgical électrique à courant continu selon l'une des revendications précédentes, **caractérisé en ce que** le boîtier d'alimentation à découpage (40 ; 140)
a) présente une ouverture de boîtier d'alimentation à découpage (62 ; 162) par laquelle l'agencement de circuit d'alimentation à découpage (68 ; 168) peut être introduit dans le logement de circuit (42 ; 142), et **en ce que** le boîtier d'alimentation à découpage (40 ; 140) comprend un couvercle de boîtier d'alimentation à découpage (60 ; 160) pour la fermeture de l'ouverture de boîtier d'alimentation à découpage (62 ; 162)
et/ou
b) est stérilisable à la vapeur
et/ou
c) est fabriqué à partir d'au moins une matière synthétique.

11. Outil chirurgical électrique à courant continu selon l'une des revendications précédentes, **caractérisé en ce que** le au moins un joint d'étanchéité (64 ; 164)
a) est disposé sur le boîtier d'alimentation à découpage (40 ; 140) en entourant l'ouverture de boîtier d'alimentation à découpage (62 ; 162)
et/ou
b) est disposé de telle manière qu'il étanchéifie le boîtier d'alimentation à découpage (40 ; 140) et le couvercle de boîtier d'alimentation à découpage (60 ; 160) l'un par rapport à l'autre quand le couvercle de boîtier d'alimentation à découpage (60 ; 160) ferme l'ouverture de boîtier d'alimentation à découpage (62 ; 162).

12. Outil chirurgical électrique à courant continu selon l'une des revendications précédentes, **caractérisé en ce qu'**il est prévu des premiers contacts électriques (66 ; 166 ; 266 ; 366) sur le boîtier d'alimentation à découpage (40 ; 140 ; 240 ; 340) pour la liaison de l'agencement de circuit d'alimentation à découpage (68 ; 168 ; 268 ; 368) avec le dissipateur électrique à courant continu (12).

13. Outil chirurgical électrique à courant continu selon la revendication 12, **caractérisé en ce que** les premiers contacts électriques (66 ; 166) sont disposés sur le couvercle de boîtier d'alimentation à découpage (60 ; 160).

14. Outil chirurgical électrique à courant continu selon l'une des revendications précédentes, **caractérisé en ce qu'**il est prévu des seconds contacts électriques (48 ; 148 ; 248 ; 348) sur le boîtier d'alimentation à découpage (40 ; 140 ; 240 ; 340) pour la liaison avec des troisièmes contacts électriques d'un conducteur de raccordement au réseau (52 ; 190) ou pour la liaison avec des quatrièmes contacts électriques (184 ; 184') d'un couvercle de boîtier (180 ; 180') de l'outil électrique à courant continu (10).

15. Outil chirurgical électrique à courant continu selon l'une des revendications précédentes, **caractérisé en ce que** l'agencement de circuit d'alimentation à découpage (268 ; 368)
a) est stérilisable
et/ou
b) est coulé en une matière synthétique stérilisable (392),
où en particulier la matière synthétique (392) est une résine époxyde,
et/ou
c) l'agencement de circuit d'alimentation à découpage (268 ; 368) et le boîtier d'alimentation à découpage (240 ; 430) sont reliés l'un à l'autre de manière inamovible.
